(19) Europäisches Patentamt
European Patent Office
Office européen des brevets

(11) EP 0 544 886 B1

(12) **EUROPÄISCHE PATENTSCHRIFT**

(45) Veröffentlichungstag und Bekanntmachung des Hinweises auf die Patenterteilung:
**10.09.1997 Patentblatt 1997/37**

(21) Anmeldenummer: **92913646.3**

(22) Anmeldetag: **24.06.1992**

(51) Int Cl.[6]: **C07H 15/244**, A61K 35/78

(86) Internationale Anmeldenummer:
**PCT/EP92/01428**

(87) Internationale Veröffentlichungsnummer:
**WO 93/00350 (07.01.1993 Gazette 1993/02)**

# (54) VERFAHREN ZUR GEWINNUNG DER SENNOSIDE A, B UND A1

METHOD OF EXTRACTING SENNOSIDES A, B and A1

PROCEDE D'EXTRACTION DES SENNOSIDES A, B et A1

(84) Benannte Vertragsstaaten:
**AT BE CH DE DK ES FR GB GR IT LI LU MC NL SE**

(30) Priorität: **25.06.1991 DE 4120991**

(43) Veröffentlichungstag der Anmeldung:
**09.06.1993 Patentblatt 1993/23**

(73) Patentinhaber: **MADAUS Aktiengesellschaft**
**D-51075 Köln (DE)**

(72) Erfinder:
- **CARCASONA, Alfons**
  **D-5000 Köln 80 (DE)**
- **GRIMMINGER, Wolf**
  **D-5060 Bergisch-Gladbach 1 (DE)**
- **HIETALA, Pentti**
  **SF-00660 Helsinki 66 (FI)**
- **ZAESKE, Helga**
  **D-5063 Overath 3 (DE)**
- **WITTHOHN, Klaus**
  **D-5063 Overath 3 (DE)**

(56) Entgegenhaltungen:
**DE-A- 3 200 131** **FR-A- 2 594 337**
**GB-A- 555 450** **GB-A- 1 135 528**
**GB-A- 2 018 488**

- **Fortschritte der Chemie organischer Naturstoffe, Band. 599, 1950 A. Stoll et al.: "Sennosides A and B, the Active Principles of Senna ", see page 248 - 269**

Bemerkungen:
Die Akte enthält technische Angaben, die nach dem Eingang der Anmeldung eingereicht wurden und die nicht in dieser Patentschrift enthalten sind.

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

EP 0 544 886 B1

## Beschreibung

Die Erfindung betrifft ein Verfahren zur Gewinnung der Sennoside A, B und A1, die im wesentlichen frei von den Sennosiden C, D und D1 und von Aloeemodinkomponenten sind sowie die nach diesen Verfahren erhältlichen Sennoside und pharmazeutische Mittel, die diese Sennoside enthalten.

Die Sennoside sind laxativ wirkende Substanzen, die in getrockneten Drogen der Gattungen Cassia und Rheum vorkommen. Die Sennadroge besteht aus den getrockneten Blättern und Schoten der Sennapflanze, beispielsweise der indischen Senna (Cassia acutifolia).

Die laxativ aktiven Sennoside sind von Rhein und Aloeemodin abgeleitete Dianthronglucoside. Die wichtigsten sind die Sennoside A, B, A1, C, D und D1. Sie entsprechen der Formel:

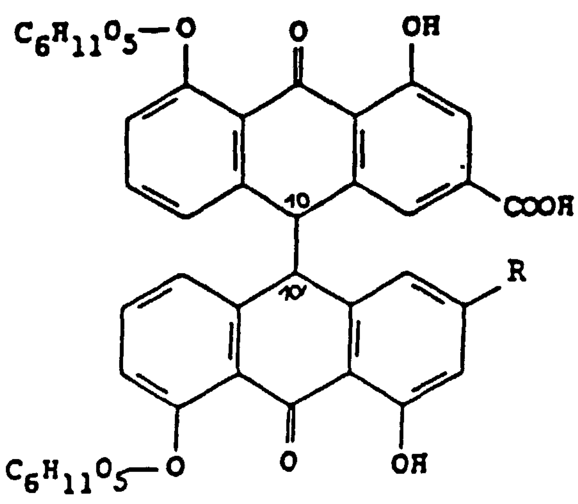

Bei den Sennosiden A, B und A1 steht R für COOH und bei den Sennosiden C, D und D1 steht R für $CH_2OH$. Die Sennoside A, B und A1 bzw. C, D und D1 sind Stereoisomere und unterscheiden sich untereinander durch die Konfiguration an den C-Atomen 10 und 10'.

Die Rohdroge enthält neben den Sennosiden auch Aglykone (Sennidine), halbglykosidierte Sennidine, Polymere, Abbauprodukte der Sennoside, Aloeemodin und Derivate davon etc., die unerwünschte Nebenwirkungen, wie Unwohlsein, Erbrechen, Blähungen und Koliken hervorrufen können.

Verfahren zur Gewinnung der Sennoside aus der Sennadroge sind beispielsweise beschrieben in DE-B-16 17 667, FR-M 6611, GB-A-832017 und DE-A-3 200 131. Die nach diesen bekannten Verfahren gewonnenen Sennoside enthalten je nach Droge ein Sennosidgemisch mit 1,5 - 5 % der Sennoside C, D und D1. Wie oben schon gezeigt, enthalten diese in ihrem Molekül einen von Aloeemodin der Formel:

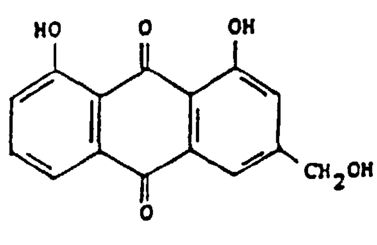

abgeleiteten Anteil. Es wäre wünschenswert, wenn man Sennoside gewinnen könnte, die im wesenlichen frei von Sennosiden C, D und D1 sind.

Eine im wesentlichen vollständige Abtrennung der Sennoside C, D und D1 aus einem Sennosidgemisch ist nach dem derzeitigen Stand der Technik nicht bekannt.

Der Erfindung liegt daher die Aufgabe zugrunde, ein Verfahren zur Gewinnung der Sennoside A, B und A1 zur Verfügung zu stellen, die im wesentlichen frei sind von unerwünschten Begleitstoffen, insbesondere von den Sennosiden C, D und D1.

Diese Aufgabe wird gelöst duch das erfindungsgemäße Verfahren, das dadurch gekennzeichnet ist, daß man

A) ein Sennosidgemisch einer Reduktion zu Rhein-9-anthron-8-glucosid unterwirft,

B) eine Flüssig-Flüssig-Verteilung der erhaltenen Verbindungen zwischen einem nur teilweise mit Wasser mischbaren polaren organischen Lösungsmittel und einer wäßrigen Phase vornimmt, und

C) das nach der Verteilung in der wäßrigen Phase enthaltene Rhein-9-anthron-8-glucosid zu den Sennosiden A, B und A1 oxidiert und diese gewinnt.

Stufe A

Als Ausgangsmaterial für das erfindungsgemäße Verfahren werden im allgemeinen Sennosidgemische eingesetzt, wie sie bei der Extraktion der Sennadroge nach den oben erwähnten Verfahren anfallen. Beispielsweise ist als Ausgangsmaterial ein Sennosidgemisch brauchbar, das man nach dem in der DE-A- 32 00 131 beschriebenen Verfahren erhält. Danach extrahiert man zunächst die Sennadroge mit wäßrigem Methanol. Das nach vollständigem Entfernen des Methanols verbleibende Konzentrat enthält die Sennoside in Form des Kaliumsalzes. Dieses Konzentrat kann als Ausgangsmaterial für das erfindungsgemäße Verfahren eingesetzt werden.

Man kann das Konzentrat noch durch Flüssig-Flüssig-Extraktion mit in Wasser teilweise löslichen Alkoholen oder Ketonen (z. B. Butanol-2, 2-Butanon, Aceton) (Raffinat) reinigen. Das Raffinat wird auf einen pH von etwa 1,5 bis 2,0 angesäuert und die Sennoside werden unter Animpfen zur Kristallisation gebracht. Das erhaltene Rohsennosidgemisch ist ebenfalls als Ausgangsprodukt für das erfindungsgemäße Verfahren brauchbar. Gewünschtenfalls kann man das Rohsennosidgemisch auch noch umkristallisieren.

Alternativ kann man das mit einem in Wasser teilweise löslichen Alkohol oder Keton, insbesondere Butanol-2, versetzte Konzentrat als Ausgangsmaterial verwenden.

Bei der Extraktion der Sennadroge beträgt das Verhältnis von Droge zu Extraktlösungsmittel vorzugsweise 1:4 bis 1:15, insbesondere 1:4 bis 1:10.

Die Extraktion wird vorzugsweise in Gegenwart eines Puffers durchgeführt, beispielsweise Trinatriumcitrat, Glycin, Natriumbicarbonat oder Saccharose.

Nach dem erfindungsgemäßen Verfahren werden diese Ausgangsmaterialien einer vollständigen Reduktion zu dem entsprechenden Rhein-9-anthron-8-glucosid (R=COOH) und dem entsprechenden Aloeemodin-9-anthron-8-glucosid ($R=CH_2OH$) der Formel:

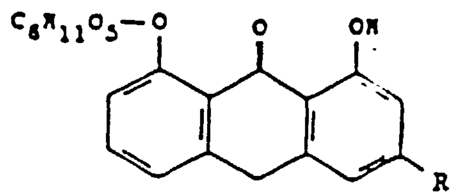

unterworfen.

Reduktionsmittel mit einem geeigneten Reduktionspotential sind Zinn-II-chlorid, Schwefeldioxid, Alkalimetallborwasserstoffe und vorzugsweise Alkalimetalldithionite, insbesondere Natriumdithionit.

Zur Durchführung der Reduktion kann man das Ausgangsmaterial in wäßriger Lösung oder Suspension vorlegen und das Reduktionsmittel in fester Form oder in Wasser gelöst zugeben. Man kann auch, insbesondere bei Verwendung von Sennesfrüchteprimärextrakt, nach der DE-A-32 00 131 (= wäßriges Konzentrat) in einem 2-Phasengemisch arbeiten, indem man ein mit Wasser teilweise mischbares, polares organisches Lösungsmittel, insbesondere 2-Butanol oder Aceton zugibt.

Man kann bei Umgebungstemperatur oder höherer Temperatur reduzieren. Die Reduktion wird zweckmäßigerweise bei 40 bis 60 °C, insbesondere bei 50 bis 55 °C, durchgeführt. Man arbeitet bei schwach saurem bis schwach alkalischem pH-Wert der Ausgangssennosidlösung zw. -Suspension, vorzugsweise bei pH-Wert 5 - 10,5. Gewünschtenfalls kann man die Reduktion mehrfach, insbesondere zwei bis zehnmal, durchführen.

Die gebildeten 9-Anthron-8-glucoside fällt man durch Zugabe einer Säure, beispielsweise Schwefelsäure, bis etwa pH-Wert 2 bis 4,5 aus. Die Temperatur sollte dabei zweckmäßigerweise nicht mehr als 40 °C betragen. Zweckmäßigerweise arbeitet man bei der Ausfällung der Anthronglucoside und bei ihrer Isolierung (beispielsweise durch Filtration) unter Stickstoff, um eine unkontrollierte Oxidation dieser Verbindungen zu vermeiden.

Es ist wesentlich, daß die Reduktion vollständig verläuft. Zweckmäßigerweise verwendet man daher das Reduktionsmittel in großem Überschuß. Dithionite, insbesondere Natriumdithionit, verwendet man im allgemeinen in der 1 bis 4fachen Gew.-Menge, bezogen auf den Gehalt des Ausgangsmaterials an Sennosiden. Außerdem läßt man das Reduktionsmittel mindestens 2 h, vorzugsweise mindestens 3 h, einwirken. Im allgemeinen erfolgt die Reduktion nicht länger als 10 h. Vorzugsweise führt man eine Nachreduktion unter den genannten Bedingungen durch.

Das erhaltene Produkt wird vor seinem Einsatz in Stufe B vorzugsweise umgefällt, indem man es in wäßriger Lösung durch Zugabe einer Base (NaOH, KOH) bis etwa pH-Wert 6 - 7 in Lösung bringt, die wäßrige Lösung mit 2-Butanol, 2-Butanon oder Aceton extrahiert und das Produkt durch Zugabe einer Säure bis etwa pH-Wert 2-4 wieder ausfällt.

Stufe B

In dieser Stufe werden die Aloeemodinkomponenten, insbesondere das Aloeemodin-9-anthron-8-glucosid, entfernt. Hierfür wird eine Flüssig-Flüssig-Verteilung des erhaltenen Produktes in einem mit Wasser nur teilweise mischbaren polaren organischen Lösungsmittel und einer wäßrigen Phase vorgenommen. Geeignete polare organische Lösungsmittel sind $C_4$-$C_5$-Alkanole und Di-$C_1$-$C_3$-Alkylketone, wie 1-Butanol, 2-Butanol, 2-Butanon und Aceton. Vorzugsweise verwendet man 2-Butanol oder Aceton.

Vorzugsweise gibt man zu der wäßrigen Phase ein Reduktionsmittel, um der wäßrigen Phase während der gesamten Flüssig-Flüssig-Verteilung ein Redox-Potential von -210 mV oder negativer zu verleihen. Zweckmäßigerweise verwendet man das gleiche Reduktionsmittel wie in Stufe A. Bei Verwendung eines Alkalimetalldithionits als Reduktionsmittel ist im allgemeinen eine 2 bis 4 Gew.-%ige Lösung bei einem pH-Wert von 7 bis 10,5 ausreichend, um die erwähnten Potentialbedingungen einzuhalten. Der pH-Wert wird zweckmäßigerweise durch Zugabe eines Puffers in diesem Bereich gehalten.

Das Volumenverhältnis von wäßriger Phase (schwere Phase) zu organischer Phase (leichte Phase) liegt im allgemeinen im Bereich von 1:5 bis 1:40.

Vorzugsweise erfolgt die Flüssig-Flüssig-Extraktion im Gegenstrom. Das Gemisch der Anthronverbindungen wird dabei in Form der nach der Reduktion erhaltenen Lösung oder, wenn die Anthronverbindungen isoliert wurden, in Form einer 3 bis 15 Gew.-%igen Lösung zugeführt.

Nach der Verteilung befindet sich das gewünschte Rhein-9-anthron-8-glucosid in der wäßrigen Phase. Es wird durch Zugabe einer Säure bis zu einem pH-Wert von etwa 2 bis 4 gefällt und in üblicher Weise gewonnen.

Stufe C

In dieser Stufe wird das Rhein-9-anthron-8-glucosid wieder zu den ensprechenden Sennosidverbindungen oxidiert. Geeignete Oxidationsmittel hierfür sind Wasserstoffperoxid, Mangandioxid, Permanganat, Mangan-III-acetonylacetonat.

Vorzugsweise führt man die Oxidation jedoch mit Sauerstoff durch. Als Sauerstoffquelle kann beispielsweise Luft dienen.

Da das Rhein-9-anthron-8-glucosid in Wasser unlöslich ist, wird es für die Oxidation in eine lösliche Form überführt. Dies geschieht beispielsweise dadurch, daß man es durch Zugabe einer geeigneten Base bis zu einem pH-Wert von etwa 6 - 7 in ein Alkalimetallsalz oder das Calciumsalz überführt. Gewünschtenfalls kann der Lösung eine geringe Menge (bis zu etwa 30 Volumen-%) eines mit Wasser nur teilweise mischbaren Lösungsmittels, insbesondere 2-Butanol, zugesetzt werden.

Die Oxidation wird in möglichst konzentrierter Lösung durchgeführt, weil auf diese Weise die Bildung der gewünschten Sennoside favorisiert wird. Zweckmäßigerweise führt man die Oxidation mit einer Lösung durch, die etwa 250 bis 300 g Rhein-9-anthron-8-glucosid pro Liter Lösungsmittel enthält. Bei Verwendung von Sauerstoff als Oxidationsmittel leitet man diesen zweckmäßigerweise durch die Lösung hindurch.

Die Oxidation mit Sauerstoff kann man durch Verwendung eines Katalysators erleichtern. Geeignete Katalysatoren sind z. B. Palladiumschwarz oder Eisen-III-Salze, insbesondere Eisen-III-chlorid. Die Menge an Katalysator liegt im allgemeinen im Bereich von 0,2 bis 2 Gew.-%, bezogen auf die Menge an Rhein-9-anthron-8-glucosid, und insbesondere im Bereich von 0,5 bis 1 Gew.-%.

Alternativ kann man die Oxydation mit Eisen-III-Salzen, z. B. $Fe_2(SO_4)_3$ oder $FeCl_3$ bei einem pH-Wert von 8 - 8,5 durchführen. Vorzugsweise arbeitet man dabei bei 30 - 50 °C und in Gegenwart von Trinatriumcitrat.

Die Oxidation wird so lange durchgeführt, bis kein Rhein-9-anthron-8-glucosid mehr nachweisbar ist. (Fehlen der UV-Fluoreszenz der Anthronverbindungen).

Die Sennoside werden durch Ansäuern der erhaltenen Lösung in üblicher Weise gewonnen. Zweckmäßigerweise

verdünnt man die Lösung vor Zugabe einer Säure mit dem zur Anwendung gekommenen Lösungsmittel (z. B. Wasser/2-Butanol) auf das 2- bis 3fache des vorhandenen Volumens. Auf diese Weise wird erreicht, daß das als Nebenprodukt gebildete Rhein-8-glucosid beim Ausfällen der Sennoside weitgehend in Lösung bleibt.

Die Abtrennung des Rhein-8-glucosids kann auch über die Calciumsalze erfolgen, weil das Calciumsalz des Rhein-8-glucosids unlöslich ist und ausfällt, während das Calciumsalz der Sennoside in Lösung bleibt.

Die Sennoside werden durch Zugabe einer Säure bis zu einem pH-Wert von etwa 2 bis 4 ausgefällt und dann in üblicher Weise gewonnen.

Bei den erhaltenen Sennosiden handelt es sich im wesentlichen um die Sennoside A, B und A1. Sie sind im wesentlichen frei von den Sennosiden C, D und D1 und anderen Aloeemodin-Verunreinigungen. Der Gehalt an den Sennosiden C, D und D1 in dem erfindungsgemäß erhältlichen Produkt beträgt weniger als 100 ppm (bestimmt gemäß der in den Beispielen angegebenen Analysenmethode).

Die Erfindung betrifft auch das erfindungsgemäß erhältliche Gemisch der Sennoside A, B und A1 sowie ein pharmazeutisches Mittel, das das erwähnte Gemisch enthält.

Das Anwendungsgebiet, die zu verabreichende Dosis und geeignete Dosierungsformen sind aus den eingangs erwähnten Publikationen bekannt.

Die nachfolgenden Beispiele erläutern die Erfindung.

**Beispiel 1**

Gewinnung des als Ausgangsmaterial verwendeten Sennosidgemisches:

Man gibt jeweils 40 kg Sennadroge in zwei in Reihe geschalteten Perkolatoren mit einem Volumen von 250 l und bedeckt sie mit einer gelochten Stahlplatte.

Als Lösungsmittel für die Extraktion verwendet man 70%iges Methanol, das auf die Droge im ersten Perkolator geleitet wird. Die im ersten Perkolator gebildete Lösung leitet man auf die Droge, die sich im zweiten Perkolator befindet. Dabei läßt man das Lösungsmittel frei durch den ersten Perkolator fließen.

Für die Extraktion von 40 kg Sennadroge verwendet man insgesamt 160 l Lösungsmittel. Nachdem man dieses Volumen 70%iges Methanol durch die beiden Perkolatoren geleitet und die entsprechende Menge Perkolat aufgefangen hat, koppelt man den Entleerungsschlauch des Perkolators mit einem Nachperkolatbehälter und leitet noch zusätzlich 60 l 70%iges Methanol durch die Perkolatoren. Danach leitet man das restliche freie Lösungsmittel aus dem ersten Perkolator in den oberen Teil des zweiten Perkolators und sammelt das Nachperkolat, bis es insgesamt 120 l ausmacht. Dann entleert man den ersten Perkolator, füllt ihn erneut mit 40 kg Sennadroge und pumpt das Nachperkolat auf die Droge, wobei 120 l Nachperkolat ausreichen, um die Droge im Perkolator zu bedecken. Anschließend bringt man die Temperatur der Lösung auf +30 °C. Danach läßt man über Nacht stehen.

Man verbindet diesen Perkolator mit dem zuvor extrahierten und führt die Extraktion wie oben beschrieben aus.

Für jeweils 40 kg Droge sammelt man 160 l Perkolat, aus dem man das Methanol in einem Vakuumrotationsverdampfer, der mit einer Füllkörpersäule ausgestattet ist, entfernt. Man erhält ca. 30 l Bodenprodukt.

Dieses Konzentrat wird mit dem gleichen Volumen 2-Butanol, das mit Wasser gesättigt ist, extrahiert. Die Phasen werden getrennt und die wäßrige Phase wird weiterverarbeitet.

Stufe A:

<u>Reduktion der Sennoside zu Rhein-9-anthron-8-glucosiden</u>

1,0 1 des extrahierten Konzentrats werden mit 48 %iger Natronlauge auf pH-Wert 7,5 gebracht. Man erhitzt auf 60 °C und gibt unter Rühren während einer halben Stunde 90 g Natriumdithionit in fester Form in die Lösung. Nach beendeter Zugabe rührt man eine weitere Stunde. Anschließend gibt man unter Rühren konzentrierte Schwefelsäure bis zu einem pH-Wert von 2 zu. Man kühlt im Laufe von zwei Stunden auf Umgebungstemperatur, filtriert den ausgefallenen kristallinen Niederschlag ab und wäscht ihn mit schwefeldioxidhaltigem Wasser.

Gewünschtenfalls wird das rohe Rhein-9-anthron-8-glucosid umgefällt. Der noch feuchte Filterkuchen wird in einer Mischung von 15 Vol.-Teilen 2-Butanol und 85 Vol.-Teilen Wasser, die 0,5 Gew.-% Natriumpyrosulfit enthält, so gelöst, daß man durch Zugabe von 48%iger Natriumhydroxidlösung bis pH-Wert 7 eine 10%ige Lösung (G/V) erhält. Die Lösung wird mit konzentrierter Salzsäure auf pH 2,8 oder darunter angesäuert und während 2 h stehengelassen. Der ausgefallene Niederschlag wird abfiltriert und mit schwefeldioxid- oder natriumpyrosulfithaltigem Wasser gewaschen und getrocknet.

Ausbeute 90 %.

Mit dem auf diese Weise erhaltenen Produkt führt man eine erneute Reduktion (Nachreduktion) wie folgt durch:

3,0 g des rohen getrockneten Rhein-9-anthron-8-glucosids oder die entsprechende Menge des feuchten Produkts löst man zusammen mit 1,4 g Natriumdithionit und 2,3 ml 5N NaOH in 15 ml Wasser. Anschließend füllt man mit Wasser auf 24 ml auf und erwärmt die Lösung 20 Min. bei 55 °C. Danach gibt man weitere 1,5 g Natriumdithionit in die Lösung und erwärmt 20 Min. auf 55 °C. Anschließend gibt man 0,9 ml 5N NaOH und 1,5 g Natriumdithionit zu. Nach 20minütigem Erwärmen auf 55 °C gibt man noch einmal 0,9 ml 5N NaOH. Die erhaltene Lösung wird direkt in die nachfolgende Flüssig-Flüssig-Extraktion eingeführt.

Stufe B:

Abtrennung der Aloeemodin-Komponenten

Die Abtrennung der Aloeemodin-Komponenten erfolgt durch Flüssig-Flüssig-Verteilung der 9-Anthron-8-glucoside im Gegenstrom mit Hilfe einer Apparatur aus 60 Mischer-Scheider-Einheiten (Mixer-Settler-Apparatur). Als wäßrige, schwere Phase verwendet man eine Lösung von 3,0 g Natriumdithionit in 3,5 ml 5N NaOH und 96 ml Wasser. Als organische, leichtere Phase verwendet man (wassergesättigtes) 2-Butanol oder Aceton. Die beiden Phasen werden so durch die Apparatur geschickt, daß das Volumenverhältnis von schwerer Phase zu leichter Phase 1:10 beträgt.

Das aufzutrennende Gemisch wird in Form der frisch reduzierten Lösung oder in Form einer Lösung von entsprechendem pH-Wert und von entsprechender Konzentration, welche die aus Stufe A erhaltenen 9-Anthron-8-glucoside enthalten, der Apparatur zugeführt, und zwar derart, daß pro Volumenteil des aufzutrennenden Gemisches 30 Vol.-Teile der organischen Phase verwendet werden.

Der pH-Wert der das Gemisch enthaltenden Lösung wird mit Hilfe eines Glycinpuffers bei 9 - 9,5 gehalten. Der Puffer aus 3 Vol.-Teile einer 7,5%igen Glycinlösung 1 Vol.-Teil 1N NaOH wird in einer Menge von 240 ml Pufferlösung pro 150 g rohes Rhein-9-anthron-8-glucosid zugegeben. Die unerwünschten Aloeemodinverbindungen reichern sich in der organischen Phase an, während das Rhein-9-anthron-8-glucosid in der wäßrigen Phase verbleibt. Die wäßrige Phase wird mit Schwefelsäure bis pH-Wert 2,8 angesäuert, der gebildete Niederschlag wird abfiltriert und mit Wasser und Aceton gewaschen und an der Luft bei Umgebungstemperatur getrocknet. Man erhält auf diese Weise Rhein-9-anthron-8-glucosid mit einem Gehalt an Aloeemodinkomponenten von 49 ppm (bestimmt als Aloeemodin).
Ausbeute: 97 %, bezogen auf Rhein-9-anthron-8-glucosid.

Stufe C:

Oxidation der Rhein-9-anthron-8-glucoside

18,8 g des erhaltenen Rhein-9-anthron-8-glucosids löst man in 56 ml Wasser und 11 ml 2-Butanol, wobei man 17N NaOH bis zu einem pH-Wert von 6,5 zugibt. Durch diese Lösung wird in einem zylinderförmigen Gefäß mit Hilfe einer Glasfritte während 5 Std. unter Rühren Luft durchgeblasen. Die Strömungsgeschwindigkeit der Luft beträgt 40 ml/Min. Der Verlauf der Oxidation wird mittels HPLC verfolgt.

Wenn kein Rhein-9-anthron-8-glucosid mehr nachweisbar ist, wird die Lösung auf ca. 200 ml mit Wasser/Butanol 56/11 verdünnt. Man gibt konzentrierte Salzsäure bis pH-Wert 1,5 bis 2,0 zu, rührt 2 Std. bei Umgebungstemperatur, filtriert die ausgefallenen Kristalle ab und wäscht sie mit Wasser und Aceton und trocknet. Man erhält 14,4 g (76 %) reines Sennosidgemisch mit einem Gehalt von 41 ppm Aloeemodinkomponenten (bestimmt als Aloeemodin) nach dem in Stufe C, Beispiel 2, angegebenen Analyseverfahren.

**Beispiel 2**

Man wiederholt das in Beispiel 1 beschriebene Verfahren, wobei man jedoch die Oxidation in Stufe C wie folgt durchführt:
150 g der reinen Rhein-9-anthron-8-glucoside und 75 g Eisen-III-chlorid-hexahydrat werden in 480 ml Wasser und 120 ml 2-Butanol gelöst. Es wird 48%ige Natriumhydroxidlösung zugegeben bis pH 6,5 erreicht und das Rhein-9-anthron-8-glucosid gelöst ist. Die Lösung wird in ein Gefäß mit einer Sinterbodenplatte gegeben. Anschließend wird ein lebhafter Luftstrom durch die Lösung geleitet. Die Oxidation ist nach ca. 30 Min. beendet. Anschließend verdünnt man die Lösung mit einem Gemisch aus 120 ml 2-Butanol und 480 ml Wasser, gibt 7,5 g Natriumdithionit zu und stellt den pH-Wert der Lösung durch Zugabe von konzentrierter Salzsäure auf 2,0 ein. Die Lösung wird 18 Std. gerührt. Anschließend wird der ausgefallene Niederschlag abfiltriert, mit 600 ml Wasser und 800 ml Aceton gewaschen und getrocknet. Der Gehalt des Produkts an Anthranoid-Verbindungen liegt zwischen 94 und 95 %.

Das Produkt wird in 200 ml 2-Butanol aufgenommen und mit 800 ml Wasser unter Zusatz von 5,5 g Natriumpyrosulfit gefällt. Nach Abfiltrieren und Trocknen des ausgefallenen Niederschlags erhält man 95,4 g eines Produkts aus Anthranoid-Verbindungen folgender Zusammensetzung (nach HPLC, Analyse eins typischen Versuchs):

| | |
|---|---|
| Rhein-8-glucosid | 1,5 % |
| Sennosid B | 49,7 % |
| Sennosid A1 | 13,3 % |
| Sennosid A | 33,6 % |
| Sennidin Monoglucoside | 1,1 % |
| Rhein | 0,02 % |
| | 99,22 % |

Sennoside C und D und Aloeemodinglucosid konnten mittels HPLC nicht nachgewiesen werden. Der Gesamtgehalt an Aloeemodin und dessen Derivaten wurde nach der folgenden Methode zu 30 ppm bestimmt.

Die Sennoside C, D und Aloeemodin-8-glucosid lassen sich im ppm-Bereich mittels HPLC-Chromatographie nicht mehr zuverlässig als Sennoside bestimmen. Es ist daher erforderlich, die zu untersuchendende Substanz durch Oxidation mit Eisen-III-chlorid unter gleichzeitiger Hydrolyse mit Salzsäure in einem 2-Phasen-Gemisch aus wäßriger Lösung/Tetrachlorkohlenstoff in Rhein bzw. Aloeemodin zu überführen. Das Rhein wird dann in ein Salz überführt, so daß es in die wäßrige Phase extrahiert werden kann und das Aloeemodin in der organischen Phase mittels HPLC bestimmt werden kann. Auf diese Weise kann der Gesamtgehalt an Sennosiden C, D, Aloeemodin-8-glucosiden und anderen Aloeemodin-Komponenten, ausgedrückt als Aloeemodin, angegeben werden.

Beispiel 3

Man wiederholt die in Beispiel 1 beschriebene Extraktion der Sennadroge und die Reduktion der Sennoside. Die Nachreduktion führt man dann wie folgt durch:

Man löst 14,0 g Saccharose, 4,5 g Natriumdithionit (85 %) und 13,3 g Kaliumacetat in 133 ml Wasser und gibt 1,3 ml 48%ige Natriumhydroxidlösung und 17,3 g Kaliumcarbonat zu. Anschließend versetzt man mit 293 ml Aceton und 50 ml Wasser. Man schüttet die Mischung in einen Scheidetrichter und trennt die Phasen, wobei man 375 ml obere Phase (Acetonphase) und 130 ml untere Phase erhält.

In 98 ml der unteren Phase löst man 1,4 ml 48%ige Natriumhydroxidlösung und 10 g rohes Rhein-9-anthron-8-glucosid. Man erwärmt auf 45 bis 50 °C und hält 20 bis 30 Min. bei dieser Temperatur. Anschließend gibt man 1,0 ml 48%ige Natriumhydroxidlösung und 3,4 g Natriumdithionit zu und erwärmt weitere 20 bis 30 Min. auf 45 bis 50 °C. Anschließend gibt man erneut 1,0 ml 48%ige Natriumhydroxidlösung und 3,4 g Natriumdithionit zu und erwärmt 20 bis 30 Min. auf 45 bis 50 °C.

Die Abtrennung der Aloeemodin-Komponente erfolgt durch Flüssig-Flüssig-Verteilung der reduzierten Lösung im Gegenstrom gegen die oben erwähnte obere Phase (Acetonphase). Die abfließende, das Rhein-9-anthron-8-glucosid enthaltende Raffinatphase, wird auf 400 ml eingeengt und mit 20 ml Butanol-2 oder Aceton versetzt. Man gibt Salzsäure oder Schwefelsäure bis pH-Wert 4,0 - 4,2 zu. Der gebildete Niederschlag wird abfiltriert, mit 40 ml Wasser und 30 ml Aceton gewaschen und anschließend getrocknet. Die anschließende Oxidation erfolgt wie in Beispiel 2 beschrieben.

Beispiel 4

Das nach der Extraktion der Sennadroge erhaltene Konzentrat wird mit ca. 2 1 Butanol-2 versetzt. Die Reduktion des Sennesfrüchtekonzentrat/Butanol-2-Gemisches wird dann in 7 Stufen unter Stickstoff als Schutzgas durchgeführt. Nach der Reduktionsstufe I erfolgt eine Fällung des rohen Rhein-9-anthron-8-glucosids.

Reduktionsstufe I

100 l Sennesfrüchtekonzentrat/Butanol-2-Gemisch, enthaltend ca. 4 kg Sennoside, werden in einem Rührbehälter vorgelegt und mit Stickstoff überlagert. Unter Rühren werden 6 l 20%ige (w) Natronlauge, danach 350 l wassergesättigtes Butanol-2 (z. B. aus Stufe II) nacheinander zugesetzt und 15 Min. gerührt. Der Ansatz wird auf 42 - 50 °C aufgeheizt und mit 7 kg Natriumdithionit versetzt. Es wird noch 45 Min. gerührt. Der pH-Wert wid mit 20%iger (w) Natronlauge bei 7,5 - 8 gehalten. Das Reduktionspotential (gegen Ag/AgCl-Elektrode) wird erforderlichenfalls durch Natriumdithionitzugabe auf unter -630 mV gehalten.Nach Kühlung auf 30-35 °C wird mit 10%iger (w) Schwefelsäure bis pH <4 innerhalb 1,5 Std. gefällt. Die entstehende Suspension wird bei kleiner Rührgeschwindigkeit <25 °C 10 Std. gerührt. Der entstandene Niederschlag wird abfiltriert. Der Niederschlag wird in 60 l 15%igem (w) Butanol-2 suspendiert, 30 Min. bei 50 - 60 °C gerührt und anschließend filtriert. Der Rückstand wird mit 100 l demineralisiertem Wasser gewaschen. Die Rohausbeute an Rhein-9-anthron-8-glucosid, bezogen auf eingesetzte Sennoside, liegt über 82 %.

Reduktionsstufe II

3,3 kg rohes Rhein-9-anthron-8-glucosid aus Stufe I werden in einer Mischung aus 42 l demineralisiertem Wasser und 7,4 l Butanol-2 suspendiert. Mit 2 l 20%iger (w) Natronlauge und 9,9 kg Trinatriumcitrat wird die Suspension in Lösung gebracht und danach mit 3,3 kg Natriumdithionit und 350 l wassergesättigtem Butanol-2 (z.B. aus Stufe III) versetzt. Der Ansatz wird auf 42 - 45 °C aufgeheizt. Der pH-Wert wird mit 20%iger (w) Natronlauge bei 8,5 - 9 gehalten. Das Reduktionspotential (gegen Ag/AgCl-Elektrode) wird erforderlichenfalls durch Natriumdithionit-Zugabe auf unter -750 mV gehalten.

Nach einer Standzeit von 30 Min. wird die Oberphase entnommen und die Unterphase in der Stufe III weiterverarbeitet.

Reduktionsstufe III

Mit der Unterphase aus der Stufe II wird unter Zusatz der folgenden Chemikalien das in Stufe II beschriebene Reduktions-/Extraktionsverfahren wiederholt:

| | |
|---|---|
| 1,65 kg | Natriumdithionit |
| 0,8 l | 20%ige (w) Natronlauge |
| 350 l | wassergesättigtes Butanol-2 (z. B. aus Stufe IV) |

Reduktionsstufen IV - VII

Mit der Unterphase aus der jeweils vorausgegangenen Stufe wird unter Zusatz der folgenden Chemikalien das in Stufe II beschriebene Reduktions-/Extraktionsverfahren wiederholt:

| | |
|---|---|
| 0,825 kg | Natriumdithionit |
| 0,4 l | 20%ige (w) Natronlauge |
| 350 l | wassergesättigtes Butanol-2 (z.B. aus dem jeweils nachfolgenden Stufen-Gegenstromprinzip) |

Die in der Stufe VII abgetrennte Unterphase wird auf 30 - 35 °C gekühlt und das Rhein-9-anthron-8-glucosid - wie in Stufe I beschrieben - ausgefällt. Der entstandene Niederschlag wird abfiltriert und mit 100 l entmineralisiertem Wasser gewaschen. Anschließend wird mit 10 l Eisen-III-sulfatlösung (28 kg $Fe_2$ $(SO_4)_3$ in 100 l demineralisiertem $H_20$) überdeckt.

Das Rhein-9-anthron-8-glucosid wird dann, wie in Beispiel 1 oder 2 beschrieben, in die Sennoside überführt.

Beispiel 5

Die Oxidation des Rhein-9-anthron-8-glucosids kann auch nach folgendem Verfahren erfolgen:

6,0 kg filterfeuchtes Rhein-9-anthron-8-glucosid werden mit 12,6 kg Trinatriumcitrat versetzt. Diese Mischung wird in 7,0 l 1N NaOH unter heftigem Rühren gelöst und mit 0,7 l Butanol-2 versetzt. Anschließend wird mit 8,8 l Eisen-III-sulfatlösung (28 kg $Fe_2(SO_4)_3$ in 100 l demineralisiertem $H_2O$) und soviel 20%iger NaOH versetzt, daß der pH-Wert ca. 8,3 beträgt. Man läßt 3-4 Stunden bei ca. 40 °C reagieren, säuert dann mit 52%iger $H_2SO_4$ auf pH-Wert 1,8 - 2,0 an und arbeitet, wie in Beispiel 1, beschrieben auf.

Beispiel 6

Alternativ bringt man Rhein-9-anthron-8-glucosid in 50 ml Wasser durch Zugabe einer Calciumhydroxid-Saccharoselösung (hergestellt durch Suspendieren von 7,0 g Calciumhydroxid in einer Lösung von 30,0 g Saccharose in 100 ml $H_2O$ und Entfernen des ungelösten Calciumhydroxids) in Lösung. Man gibt 20 ml Butanol-2 hinzu und leitet während 90 Minuten einen lebhaften Luftstrom durch die Lösung. Man gibt 5,0 g $CaCl_2.2H_2O$ zu und stellt den pH-Wert mit der Calciumhydroxid-Saccharose-Lösung auf 8,5 ein. Der gebildete Niederschlag wird abfiltriert, und das Filtrat wid mit $H_2O$ auf 340 ml verdünnt, mit 60 ml Butanol-2 versetzt und mit konzentrierter Salzsäure auf pH-Wert 2,0 gebracht. Die weitere Aufarbeitung erfolgt wie im Beispiel 1 beschrieben.

Pharmakologische Untersuchungen

Laxative Wirkung

Der laxative Effekt des erfindungsgemäßen Sennosidgemisches wurde bei Mäusen bestimmt. Es wurden männlichen NMRI i-Mäuse verwendet, die während der Versuche in Plexiglaskäfigen gehalten wurden und Standardfuttergemisch mit Leitungswasser (1:1) in breiiger Konsistenz erhielten. Eine separate Trinkwasserzufuhr erfolgte während der Versuche nicht.

Die Tiere erhielten 100, 200 und 400 mg/kg des SennosidGemisches in 10 ml 0,5%iger $NaHCO_3$/kg per Schlundsonde.

Nach Verabreichung der zu testenden Verbindungen wurden Kot und Urin der Tiere über 24 Stunden aufgefangen und bestimmt. Die erhaltenen Ergebnisse, bezogen auf kg Körpergewicht, sind in der nachfolgenden Tabelle zusammengestellt.

Es ist ersichtlich, daß die Sennoside eine gute laxative Wirkung zeigen, die relativ schnell eintritt. Die Zeit bis zum Auftreten des ersten Weichkotes (2 Stunden) ist jedoch noch mit einem vorherigen Transit zum Dickdarm und einem Aufschluß der Sennoside durch die Dickdarmflora zu vereinbaren. Eine Dosis-Wirkungsbeziehung ist vorhanden.

Akute Toxizität:

Je 10 männlichen und weiblichen Wistar-Ratten wurden Sennoside einmalig in Dosierungen von 2.000 bis 25.000 mg/kg mit einer Schlund-Sonde zugeführt.

Substanzbedingte makroskopische Organschädigungen konnten bei den Ratten nicht beobachtet werden. Die ermittelten $LD_{50}$-Werte lauten:

**+ 840)**
**männliche Ratte: 5.200 - 720 ) mg/kg**

**+ 380)**
**weibliche Ratte: 3.530 - 340) mg/kg.**

Bei männlichen und weiblichen Mäusen (n=8, Stamm NMRI) führte die maximal applizierbare Dosis von 5.000 mg/kg zu keinen Todesfällen. Diarrhöen traten bei allen Mäusen auf, wenn auch in geringerem Ausmaß als bei den Ratten. Die $LD_{50}$-Werte betragen für beide Geschlechter > 5.000 mg/kg.

## T a b e l l e

**Laxativer Effekt des erfindungsgemäßen Sennosidgemisches bei Mäusen**

| Dosis (mg/kg) | Tierzahl | Zahl der Normalkotpellets | Zahl der Weichkotpellets | Weichkot in % der Gesamtkot-ausscheidung |
|---|---|---|---|---|
| 0 | 30 | 1265 | 0 | 0 |
| 100 | 40 | 587 | 144 | 28.0 |
| 200 | 30 | 223 | 239 | 56.0 |
| 400 | 30 | 236 | 282 | 60.0 |

## Patentansprüche

1. Verfahren zur Gewinnung der Sennoside A, B und Al der Formel:

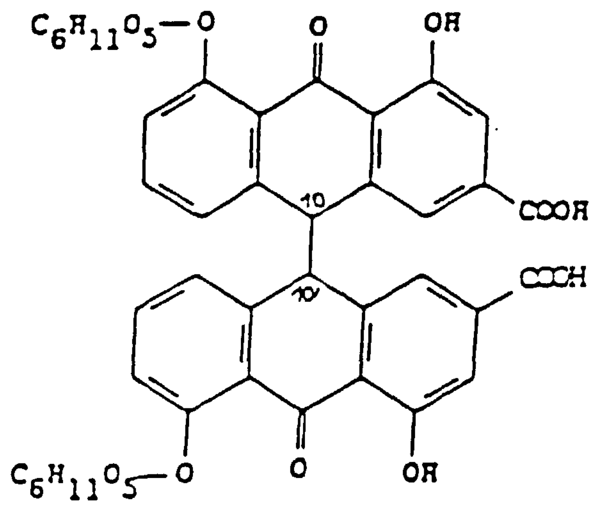

die im wesentlichen frei von den Sennosiden C, D und D1 und von Aloeemodinkomponenten sind, **dadurch gekennzeichnet,** daß man

A) ein Sennosidgemisch einer Reduktion zu Rhein-9-anthron-8-glucosid und Aloeemodin-9-anthron-8-glucosid unterwirft,

B) eine Flüssig-Flüssig-Verteilung der erhaltenen Verbindungen zwischen einem nur teilweise mit Wasser mischbaren polaren organischen Lösungsmittel und einer wäßrigen Phase durchführt und

C) das nach der Verteilung in der wäßrigen Phase enthaltene Rhein-9-anthron-8-glucosid wieder zu den entsprechenden Sennosiden oxidiert und diese gewinnt.

2. Verfahren nach Anspruch 1, **dadurch gekennzeichnet,** daß das Sennosidgemisch erhältlich ist durch Extraktion der Sennadroge mit wäßrigem Methanol, vorzugsweise in Gegenwart eines Puffers.

3. Verfahren nach Anspruch 1 oder 2, **dadurch gekennzeichnet,** daß das in Stufe A als Reduktionsmittel ein Alkalimetalldithionit verwendet.

4. Verfahren nach Anspruch 3, **dadurch gekennzeichnet,** daß das bei einem pH-Wert von 5 bis 10,5 arbeitet.

5. Verfahren nach einem der vorhergehenden Ansprüche, **dadurch gekennzeichnet**, daß man in Stufe B als polares organisches Lösungsmittel 2-Butanol verwendet.

6. Verfahren nach Anspruch 1 bis 4, **dadurch gekennzeichnet,** daß man in Stufe B als polares organisches Lösungsmittel Aceton verwendet.

7. Verfahren nach einem der vorhergehenden Ansprüche, **dadurch gekennzeichnet**, daß man in Stufe B eine wäßrige Phase verwendet, deren Redoxpotential -210 mV oder negativer ist.

8. Verfahren nach einem der vorhergehenden Ansprüche, **dadurch gekennzeichnet**, daß man die Flüssig-Flüssig-Verteilung in Stufe B im Gegenstrom durchführt.

9. Verfahren nach einem der vorhergehenden Ansprüche, **dadurch gekennzeichnet,** daß man die Oxidation in Stufe C mit Sauerstoff oder einem Eisen-(III)-Salz durchführt.

10. Verfahren nach Anspruch 9, **dadurch gekennzeichnet,** daß man die Oxidation mit Sauerstoff bei schwach saurem pH-Wert durchführt.

11. Verfahren nach Anspruch 9 oder 10, **dadurch gekennzeichnet,** daß man die Oxidation mit Sauerstoff in Gegenwart eines Katalysators, insbesondere eines Eisen-(III)-Salzes, durchführt.

12. Sennoside A, B und A1, die im wesentlichen frei von den Sennosiden C, D und D1 und von Aloeemodinkomponenten sind.

13. Pharmazeutisches Mittel, enthaltend die Sennoside nach Anspruch 12, gegebenenfalls zusammen mit üblichen pharmazeutischen Trägern und Hilfsstoffen.

## Claims

1. Process for the isolation of sennosides A, B and A1 of the formula:

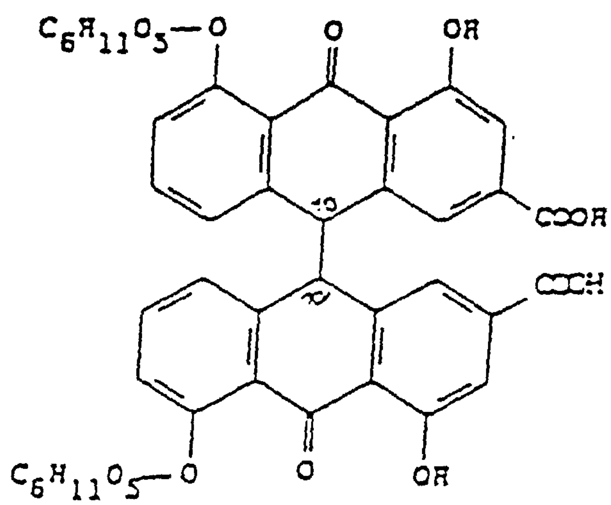

which are substantially free from the sennosides C, D and D1 and from aloe emodin components, characterised in that

A) a mixture of sennosides is subjected to a reduction to rhein-9-anthrone-8-glucoside and aloe-emodin-9-anthrone-8-glucoside,

B) a liquid-liquid partitioning of the compounds obtained is carried out between a polar organic solvent only partly miscible with water and an aqueous phase and

C) the rhein-9-anthrone-8-glucoside contained in the aqueous phase after the partitioning is reoxidised to the corresponding sennosides and these are isolated.

2. Process according to claim 1, characterised in that the mixture of sennosides is obtainable by extraction of the senna drug with aqueous methanol, preferably in the presence of a buffer.

3. Process according to claim 1 or 2,

characterised in that an alkali metal dithionite is used as reducing agent in step A.

4. Process according to claim 3, characterised in that the procedure is carried out at a pH value of from 5 to 10.5.

5. Process according to any one of the preceding claims, characterised in that 2-butanol is used as polar organic solvent in step B.

6. Process according to claims 1 to 4, characterised in that acetone is used as polar organic solvent in step B.

7. Process according to any one of the preceding claims, characterised in that in step B an aqueous phase is used, the redox potential of which is -210 mV or more negative.

8. Process according to any one of the preceding claims, characterised in that the liquid-liquid partitioning in step B is carried out in countercurrent.

9. Process according to any one of the preceding claims, characterised in that the oxidation in step C is carried out using oxygen or an iron(III) salt.

10. Process according to claim 9, characterised in that the oxidation is carried out at a weakly acid pH value using oxygen.

11. Process according to claim 9 or 10, characterised in that the oxidation is carried out using oxygen in the presence of a catalyst, in particular an iron (III) salt.

12. Sennosides A, B and A1 which are substantially free from the sennosides C, D and D1 and from aloe emodin components.

13. Pharmaceutical agent containing the sennosides according to claim 12, optionally together with conventional pharmaceutical carriers and auxiliary substances.

**Revendications**

1. Procédé pour l'extraction des sennosides A, B et A1 de la formule :

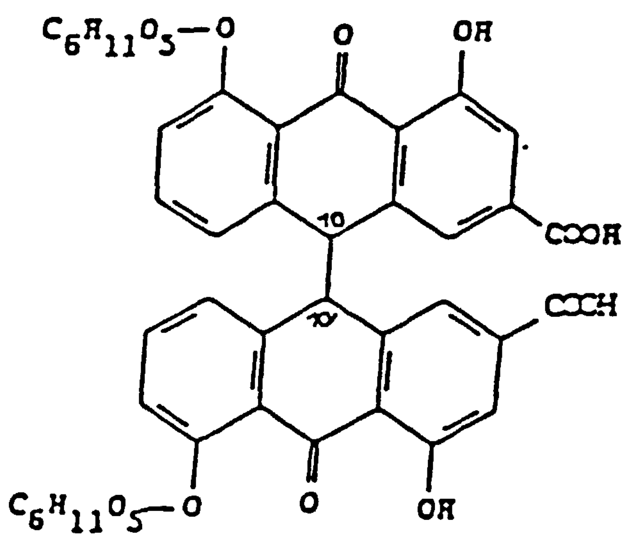

qui sont en principe essentiellement exempts des sennosides C, D et D1 et des composants aloémodine, caractérisé en ce que l'on soumet

A) un mélange de sennosides à une réduction en rhéin-9-anthron-8-glucoside et aloémodin-9-anthron-8-glucoside,
B) on procède à une répartition fluide-fluide des composés obtenus entre un solvant organique polaire qui n'est miscible que partiellement à l'eau et une phase aqueuse, et
C) le rhéin-9-anthron-8-glucoside contenu dans la phase aqueuse après la répartition est à nouveau oxydé en les sennosides correspondants et on extrait ceux-ci.

**2.** Procédé selon la revendication 1,
caractérisé en ce que le mélange de sennosides peut être obtenu par extraction de la sennadrogue avec un méthanol aqueux, de préférence en présence d'un tampon.

**3.** Procédé selon la revendication 1 ou 2,
caractérisé en ce que l'on utilise à l'étape A une dithionite des métaux alcalins en tant qu'agents de réduction.

**4.** Procédé selon la revendication 3,
caractérisé en ce que l'on travaille à une valeur de pH de 5 à 10,5.

**5.** Procédé selon l'une des revendications précédentes, caractérisé en ce que l'on utilise à l'étape B le 2-butanol en tant que solvant organique polaire.

**6.** Procédé selon les revendications 1 à 4,
caractérisé en ce que l'on utilise l'acétone à l'étape B en tant que solvant organique polaire.

**7.** Procédé selon l'une des revendications précédentes, caractérisé en ce que l'on utilise à l'étape B une phase aqueuse dont le potentiel Redox ou d'oxydoréduction est de -210 mV ou plus négatif.

**8.** Procédé selon l'une des revendications précédentes, caractérisé en ce que l'on effectue la répartition liquide-liquide à l'étape B à contre-courant.

**9.** Procédé selon l'une des revendications précédentes, caractérisé en ce que l'on procède à l'oxydation à l'étape C avec de l'oxygène ou un sel de fer (III).

**10.** Procédé selon la revendication 9,
caractérisé en ce que l'on procède à l'oxydation avec de l'oxygène à une valeur de Ph faiblement acide.

**11.** Procédé selon la revendication 9 ou 10,
caractérisé en ce que l'on procède à l'oxydation avec de l'oxygène en présence d'un catalyseur, en particulier d'un sel de fer (III).

**12.** Sennosides A, B et A1 qui sont essentiellement exempts des sennosides C, D et D1 et des composants aloémodine.

**13.** Produit pharmaceutique contenant les sennosides selon la revendication 12, éventuellement conjointement avec les véhicules pharmaceutiques habituels et des adjuvants.